# EUROPEAN PATENT SPECIFICATION

(11) **EP 2 574 664 B1**
(45) Date of publication and mention of the grant of the patent: **23.07.2014**
(21) Application number: 11460052.1
(22) Date of filing: 30.09.2011
(51) Int. Cl.: C12N 5/00, C09D 179/02, C08G 73/02

(54) **Method for preparation a thermosensitive coating substrate, the substrate with a thermosensitive coating and its application**
Verfahren zur Herstellung einer wärmeempfindlichen Beschichtung, Substrat mit wärmeempfindlicher Beschichtung und seine Anwendung
Méthode pour la préparation d'un revêtement thermosensible, substrat avec revêtement thermosensible et son utilisation

(43) Date of publication of application: 03.04.2013
(73) Proprietor: Centrum Materialów Polimerowych i Weglowych PAN, 41819 Zabrze (PL); Centrum Leczenia Oparzen, 41-100 Siemianowice Slaski (PL); Slaski Uniwersytet Medyczny, 40-055 Katowice (PL)
(72) Inventor: Utrata-Wesolek, Alicja, 47-400 Racibórz (PL); Walach, Wojciech, 41-710 Ruda Slaska (PL); Oleszko, Natalia, 42-612 Tarnowskie Góry (PL); Dworak, Andrzej, 41-800 Zabrze (PL); Trzebicka, Barbara, 44-200 Rybnik (PL); Kowalczuk, Agnieszka, 41-800 Zabrze (PL); Aniol, Jacek, 43-155 Bierun Nowy (PL); Lesiak, Marta, 43-190 Mikolów (PL); Sitkowska, Anna, 41-506 Chorzów (PL); Sieron, Aleksander L., 41-100 Siemianowice SI (PL); Kawecki, Marek, 43-384 Jaworze (PL); Glik, Justyna, 41-500 Chorzów (PL); Klama-Baryla, Agnieszka, 41-200 Sosnowiec (PL); Nowak, Mariusz, 40-820 Katowice (PL)

(56) References cited:
- EP-A1- 2 330 182
- WO-A1-2011/024963
- NAIMA A. HUTTER ET AL: "Microstructured poly(2-oxazoline) bottle-brush brushes on nanocrystalline diamond", PHYSICAL CHEMISTRY CHEMICAL PHYSICS, vol. 12, no. 17, 1 January 2010 (2010-01-01), page 4360, XP55020080, ISSN: 1463-9076, DOI: 10.1039/b923789p
- YOSHIKI CHUJO ET AL: "Synthesis of amphiphilic silane coupling agents based on poly(2-ethyl-2-oxazoline) and their reactions with tetraethoxysilane", POLYMER BULLETIN, vol. 31, 1 January 1993 (1993-01-01), pages 317-322, XP55020053, DOI: 10.1007/BF00692958
- FUKUMORI K ET AL: "Ondo Otosei Nano Graft Gel o Kochiku shita Glass Hyomen no Sakusei to Saibo Baiyo eno Oyo -Control of cell detachement on poly(N-isopropylacrylamide) grafted glass surfaces using electron beam irradiation", 54TH SPSJ SYMPOSIUM ON MACROMOLECULES : YAMAGATA, SEPTEMBER 20 - 22, 2005 = 54.-KÔBUNSHI-TÔRONKAI-YOKÔSHÛ; [POLYMER PREPRINTS], TÔKYÔ : SOCIETY OF POLYMER SCIENCE, JP, vol. 54, no. 2, 20 September 2005 (2005-09-20), pages 5221-5222, XP008146870,
- CHRISTOVA D ET AL: "New thermo-responsive polymer materials based on poly(2-ethyl-2-oxazoline) segments", POLYMER, ELSEVIER SCIENCE PUBLISHERS B.V, GB, vol. 44, no. 8, 1 April 2003 (2003-04-01), pages 2255-2261, XP004414594, ISSN: 0032-3861, DOI: 10.1016/S0032-3861(03)00139-3

## Description

The subject matter of this invention is a method for making a substrate with a thermosensitive layer, substrate with thermosensitive layer and applications for this substrate.

The material is intended for use in regenerative medicine, biology, biotechnology and other related fields.

In the treatment of severely burned patients, numerous solutions that are intended to exchange a burn wound for a surgical wound are applied to prevent infection. Skin autotransplantation is the best solution. However, this method is not always effective due to insufficient donor sites. Alternative applications of biological dressings, i.e., skin allografts or xenografts, provide only a temporary solution because in time, they are rejected by the patient's organism and introduce an opportunity for infection with zoonotic pathogens [1-3]. Skin substitutes obtained by tissue engineering may also be used. Collagen, glycosaminoglycans, polyglycolide or hyaluronic acid or biodegradable matrices [4,5] are used as skin components. Such components make up a matrix which may be covered with fibroblasts or keratinocytes obtained through in vitro culture. However, application of these skin components is quite expensive, and problems may occur in the therapeutic process. Moreover, receiving a required number of keratinocytes is time-consuming and very often results in their death thus reducing the effectiveness of the culture. A culture of a patient's own skin cells, such as keratinocytes, and their individual application to the wound is an alternative to the above methods. Nevertheless, such a procedure reveals essential deficiencies [3,6]. The first, as described above, refers to the slow culture of keratinocytes. Additionally, after cells proliferation, enzymatic methods are used to separate the cells from the substrate, but the enzymes destroy both the extracellular matrix produced by the cells and important proteins. Cell sheets are disintegrated (i.e., after detachment the cells are not linked together), and the majority of them die. Keratinocytes are then applied to the wound in the form of a suspension and create cell clusters. Thus, precise control over the arrangement of the cells to thoroughly cover a wound is not possible.

Scientific [7-10] and patent literature describes methods of cell culture on solid base substrates to which a thermosensitive polymer has been covalently attached. Such polymers and copolymers exhibit reversible changes in their properties with changes in temperature; they are soluble only below a given temperature and above that temperature (the so-called cloud point temperature, T_{CP}) they precipitate from the solution. For a substrate with a thermosensitive polymer or copolymer layer with a changeable affinity for water within the 0-80°C range, the cell culture is performed at a temperature higher than the lower critical solution temperature (LCST) of the polymer at which the surface is hydrophobic. After cells proliferation, the culture temperature is reduced below the LCST until the polymer or copolymer layer becomes hydrophilic. The cells detach from the substrate and may be removed in the form of a sheet. The application of enzymatic or mechanic methods of cell separation is not required.

The cell-culture stage is preceded by the preparation of the surface related to the modification of the base substrate followed by the attachment of the thermosensitive polymer layer.

The surfaces of the materials used as base substrates may be modified with chemical and physicochemical methods targeted to produce structural changes in the surface or changes in the wettability of the surface. Such materials are then used as base substrates to be combined with a thermosensitive polymer. In order to do this, radiation grafting or photografting are commonly applied and lead to the formation of chemical bonds between the base substrate and the polymer.

With reference to base substrates used in cell cultures, patent documents (e.g., JP 6104061 or JP5192130 and others) discuss substrates that can be made of any type of polymer (including polyethylene, polypropylene, poly(ethylene terephthalate), poly(tetrafluoroethylene), polystyrene and poly(methyl methacrylate)), ceramics, inert metals and glass. The substrate shape and type is not limited, but plates, dishes or flasks are usually used for cell culture.

Scientific and patent literature indicates that modified polystyrene (TCPS - tissue culture polystyrene) is primarily used as the base substrate. Among other patent publications (JP 2006008975 (A)), apart from polystyrene, only glass and porous poly(ethylene terephthalate) surfaces modified by the use of a coupling agent have been described for applications in cell culture. The grafting of polymers including poly(N-isopropylacrylamide) onto such a substrate has been performed in the presence of a radical initiator.

A substrate for cell culture, whose surface is covered with a thermosensitive polymer or copolymer that can reversibly alter its properties from hydrophilic to hydrophobic with changes in temperature, is the subject of patent EP0382214(B1). A poly(N-isopropylacrylamide) or poly(N,N-diethylacrylamide) layer was attached in a polystyrene Petri dish during grafting with electron beam irradiation. Endothelial tissue cells were cultured on the substrate in a cell culture medium at a temperature of 37°C and in the presence of 5% CO₂. After proliferation the culture temperature was reduced from 37°C to 4°C, and a separation of the cells was observed.

A method of skin cells culture and substrate preparation was also the subject of patent application JP5192138(A) in which N-isopropylacrylamide dissolved in isopropyl alcohol was grafted onto a Petri dish by electron beam irradiation. The culture of keratinocytes was performed on the dish, sterilised with ethylene oxide, according to the method and conditions described above.

Issues related to cell culture, including many types of cells in one layer and substrates with at least one thermosensitive polymer, were the subject of other reported inventions (e.g., WO2010010837(A1)) in which two or more polymers grafted onto a base substrate in different areas are used for cell culture. According to EP1264877A1 the cell culture is performed on the substrate covered with a layer of polymers with differing affinities to water. The base substrate may not only be polystyrene and poly(methyl methacrylate), but may also include other materials such as ceramics and metals. Similar issues are the subject of patent applications WO2010044417(A1) and EP2348099(A1).

In application EP0470681 (A1) a description and issues concerning the culture of many cell types in clusters of controllable size are presented. Invention US20080009063A1 concerns cell culture on a substrate whose surface is shaped by numerous craters that form various patterns covered with at least one thermosensitive polymer. Epidermal cells, in accordance with reported invention WO0210349(A1), may also be cultured on a substrate covered with one or many layers of a thermosensitive polymer, and the separation of the cells from the substrate is possible with the use of a polymer membrane.

A multilayered structure of the cell culture substrate, the method for its preparation and the cells cultured on it are the subject of reported invention WO2010047171 (A1). A polystyrene base substrate covered with a thermosensitive polymer (poly(N-isopropylacrylamide)) and grafted by e-beam radiation makes up the substrate layers. The thermosensitive polymer layer was then silylated and covered with anti-adhesive polyethylene glycol either on the entire surface or on selected areas.

In examples described in scientific and patent literature and in the aforementioned examples, the polymer layer of a cell culture substrate was made of a thermosensitive polymer: poly(N-isopropylacrylamide) NIPAM [8,9,12-14] and its derivatives, i.e., copolymers of NIPAM and n-butylmethacrylate [15,16], NIPAM and ethylene glycol [17-19], NIPAM and 2-carboxy isopropyl acrylamide (CIPAM) [20-22].

These polymers were primarily grafted onto substrates made of modified polystyrene (TCPS) in sophisticated apparatus using radiation grafting technology. In this process, the polymer (PNIPAM) covalent bonding to the substrate occurs with its simultaneously crosslinking and a hydrogel layer on the surface, at which chains are linked together in a network crosslinks, is created.

The polymer grafting density on the base substrate ranges from 5 to 80 µg/cm², and if the grafting density is lower than 5 µg/cm², the cells are cultured as a monolayer. However, problems with the separation of the cultured cell sheet from the substrate may occur. When the grafting density is higher than 80 µg/cm², the polymer layer is unstable, and cell adhesion may be impeded.

Terms used in the description are defined as follows:
- LCST - lower critical solution temperature
- terminating group - a functional, nucleophilic group capable of reacting with a cationic propagating center in the cationic polymerization process
- covalent bond - chemical bonding between atoms with a difference in electronegativity of up to 1.7 on the Pauling scale, e.g., C-C, C-O, C-N, etc.
- polymer brush on the surface - a surface that consists of end-tethered, grafted or anchored polymer chains that stretch away from the substrate surface
- TCPS (tissue culture polystyrene) - modified polystyrene for cell or tissue culture
- "grafting from" - type of a polymer immobilization consisting of the initiation of polymerization with an initiator grafted onto a base substrate and resulting in the linking of subsequent monomer particles to the growing polymer chain attached to the substrate
- "grafting to" - type of polymer immobilization consisting of the attaching to the base substrate the polymer chain formed earlier at the stage of polymerization initiated by an initiator in solution
- confluence - the number of cells on the surface that defines the percentage of the area occupied by the growing cells
- chromic mixture - a solution of sodium dichromate in concentrated sulfuric acid used for glass washing and degreasing
- piranha solution - a solution of concentrated sulfuric acid with 30% hydrogen peroxide used to remove surface contamination
- polymer immobilization - attachment of polymer chains to a surface without the loss of chain mobility
- cationic initiating group - positively charged group that is a part of an initiator to initiate polymer chain growth
- silyl linker - a connection between a polymer and a base substrate created in the reaction between the polymer chain end and a compound with a silicon atom and a functional group that has been previously attached to the substrate
- cationic propagating center-positively charged, reactive end-group capable of attaching monomer particles and reproducing the same center, thus causing growth of the polymer chain in a cationic polymerization reaction

A non-organic glass or silicon substrate is cleaned and modified with functional reactive groups in an argon atmosphere to be used as the base for the production of a substrate with a thermosensitive polymer layer of changeable affinity for water (LCST) within 0-80°C in accordance with this patent application. The immobilization of the thermosensitive polymer is performed by chain grafting with the formation of covalent bonds in a solution. The thermosensitive polymer immobilization is essentially performed from the solution of living homo- and copolymers from a group of 2-substituted-2-oxazoline, preferably in acetonitrile, in which covalent bonds are created in the reaction between the nucleophilic terminating groups of the base substrate (obtained as a result of substrate modification) and the cationic propagating center at the chain end of the polymers derived from the group of 2-substituted-2-oxazoline.

The immobilization is preferably performed until a polymer layer with a brush conformation and a polymer grafting density of 0.1-1.8 chain/nm² is formed on a base substrate.

The nucleophilic terminating group is preferably selected from groups including amino groups and their derivatives, hydroxyl groups and their derivatives, and thiol groups and their derivatives. The cationic initiating group is also preferably selected from a group including p-toluenesulfonic acid and its derivatives with esters, trifluoromethanesulfonic acid and its derivatives with esters, alkyl halides, benzyl halides, antimony and boron fluorides, sulfuric acid and its esters, hydrochloric acid, periodic and perchloric acid and their salts, chloroformates, and trichloroacetic acid and its derivatives.

A non-organic glass or silicon substarte is cleaned and modified with reactive functional groups in an argon protective atmosphere to be used as the base for the production of a substrate with a layer of thermosensitive polymer of changeable affinity to water (LCST) within 0-80°C in accordance with other invention variants. The thermosensitive polymer immobilization is performed in a solution by chain grafting to form covalent bonds. The immobilization of the thermosensitive polymers from a group of homo- and copolymers of 2-substituted-2-oxazoline is essentially performed in a solution, preferably of acetonitrile, in which covalent bonds are made during polymerization of the monomer from a group of 2-substituted-2-oxazoline, as described by formula (1). The polymerization is initiated by cationic initiator occurring on the base substrate that is a result of the substrate modification.

The immobilization is preferably performed until a polymer layer with a brush conformation and a polymer grafting density of 0.1-1.8 chain/nm² is formed on the base substrate.

The initiator of the cationic polymerization is preferably selected from a group that includes p-toluenesulfonic acid and its derivatives with esters, trifluoromethanesulfonic acid and its derivatives with esters, alkyl halides, benzyl halides, chloroformates, and trichloroacetic acid and its derivatives.

The content of the initiating groups should range from 10.0 to 0.08 mol percent in relation to at least one monomer from the group of 2-substituted-2-oxazoline.

The substrate consists of a non-organic glass or silicon base substrate that has been modified by the covalent bonding of a silyl linker through a functional group with the polymer layer. The polymer layer is immobilized onto the substrate during the grafting of the thermosensitive polymer or copolymer chains. Essentially, the polymer layer is comprised of a polymer or copolymer from a group of 2-substituted-2-oxazoline and is of a polymer brush conformation. The density of the chains connected to the functional group of the silyl linker is 0.1-1.8 chain/nm².

The polymer layer is preferably covalently bonded to the functional group of the silyl linker by the formula (2) element.

The polymer layer is also preferably covalently bonded to the functional group of the silyl linker by the formula (3) element.

The substrate with a thermosensitive layer composed of homo- and (co)polymers from a group of 2-substituted-2-oxazoline is then applied to the culture of cells, particularly skin cells.

Research related to thermosensitive substrates, both in the field of applied materials (which have not been fully recognized) and in the processes themselves, shows that the need for highly specialized and expensive apparatus is often a major constraint to achieving positive results.

This invention provides a solution, both in terms of producing the thermosensitive substrate and the use of new polymeric materials from a group of homo- and copolymers of 2-substituted-2-oxazoline. The present work allows the substrate to be received without application of highly specialized apparatus at the individual stages of the process, with parameters that enable cell culture - especially that of skin cells, and with the ability to separate the cell sheet by merely reducing the temperature below the LCST. The substrate, with its surface structure consisting of a polymer brush conformation, differs from the substrates with thermosensitive surfaces based on PNIPAM, which are described in the scientific and patent literature in which the polymer chains are not isolated, but linked by network crosslinks and have a hydrogel layer formed on their surface. The structure of the polymer brush conformation obtained in the method described in this patent is characterized by a quick and strong response to temperature stimulus.

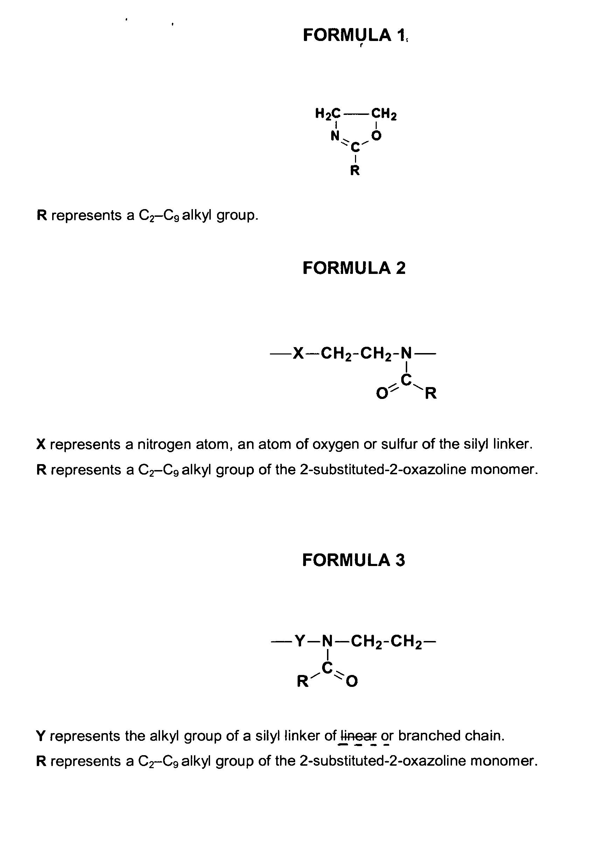

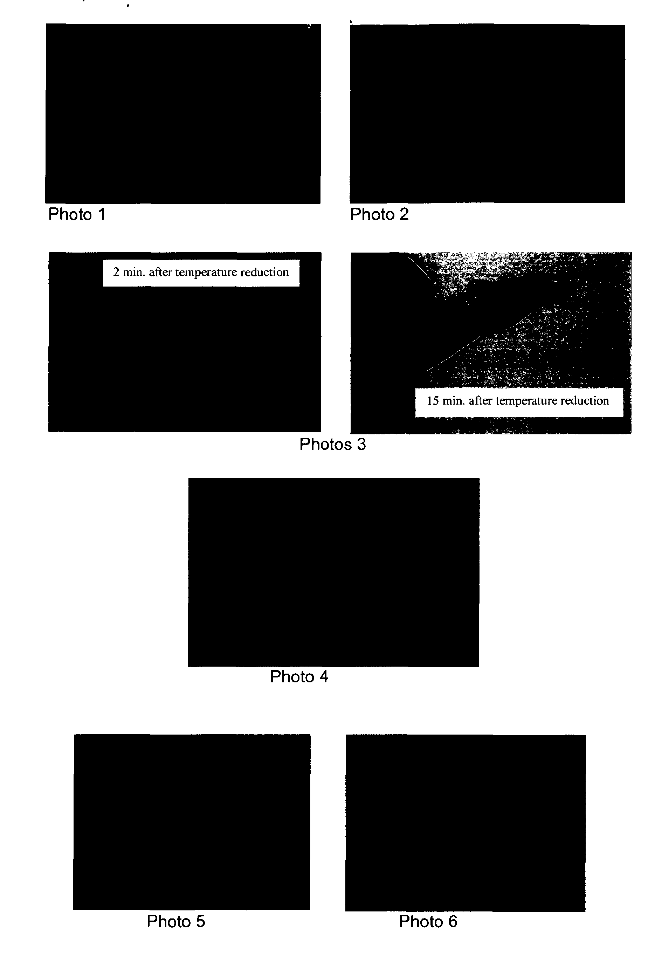

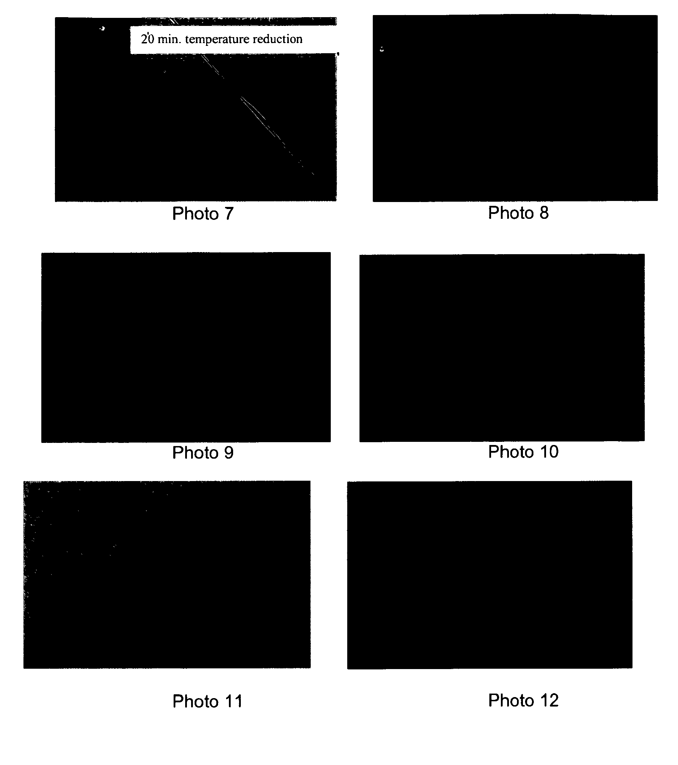

Figure 1 shows the structural formula of the monomer from a group of 2-substituted-2-oxazoline. Figures 2 and 3 show structural formulas for the bonds between the polymer layer and the functional group of the silyl linker, whereas images 1-12 present the results of cell culture on the substrates derived in accordance with this invention and on the control substrates.

The invention relates to a substrate with a layer of thermosensitive polymer with changeable affinity to water (LCST) within 0-80°C and a method for its production. The substrate preparation is carried out in several stages, including the base substrate preparation stage and later stages of the process in which the immobilization of the thermosensitive polymer onto the base substrate is performed using the "grafting from" and "grafting to" methods to create the polymer layer.

The polymer layer is synthesized on base substrate of non-organic glass or silicon containing hydroxyl functional groups that may be further modified to allow for stable covalent bonding of the base substrate with the thermosensitive polymer. For polymer immobilization onto the substrate via the "grafting to" method, functionalization occurs by the introduction of nucleophilic terminating groups including amine groups and their derivatives, hydroxyl groups and their derivatives, and thiol groups and their derivatives onto the base substrate. In the exemplary design, 3-aminopropyltriethoxysilane (APTES) was used to introduce the amine groups onto the base substrate.

In the "grafting from" method, groups that initiate cationic polymerization in the immobilization process are introduced to the base substrate. These groups include p-toluenesulfonic acid and its derivatives with esters, trifluoromethanesulfonic acid and its derivatives with esters, alkyl halides, benzyl halides, chloroformates, and trichloroacetic acid and its derivatives. In the invention exemplary design, 3-[bis(2-hydroxyethyl)amino]propyltriethoxy-silane esterified with trifluoromethanesulfonic anhydride was used to introduce the trifluoromethanesulfonic group onto the base substrate and it is essential that the content of the initiating groups with relation to at least one monomer from a group of 2-substituted-2-oxazoline amounts from 10.0 to 0.08 mol percent.

All activities connected with the substrate preparation have been performed in a laminar chamber with the use of filtered solvents and solutions to minimize the possibility of contamination.

The base substrates with modified surfaces containing a silyl linker with the above-mentioned functional groups were used for stable covalent bonding with a polymer or copolymer from a group of homo- and copolymers of 2-substituted-2-oxazoline. A controlled polymer immobilization on the surface was simultaneously conducted.

According to one invention variant, covalent bonds are formed with the substrate during the initiation of the cationic polymerization of the monomers by the initiator on the base substrate, and bonds are formed between the nitrogen atom of the 2-substituted-2-oxazoline monomer and the alkyl groups of the silyl linker. In another invention variant, the bonds are formed during termination of the growing polymer chains in the reaction with the functional groups of the silyl linker. In this variant, the bonds may occur between the nitrogen atom from the amine group, or the oxygen atom from the hydroxyl group, or the sulfur atom from the thiol group with the carbon atom from the last unit of the poly(2-substituted-2-oxazoline).

In the model solutions, poly(2-isopropyl-2-oxazoline) and a copolymer of 2-ethyl-2-oxazoline and 2-nonyl-2-oxazoline have been used to illustrate the "grafting to" method as shown in Diagram 1. Poly(2-isopropyl-2-oxazoline) was used to illustrate the "grafting from" method, as shown in Diagram 2.

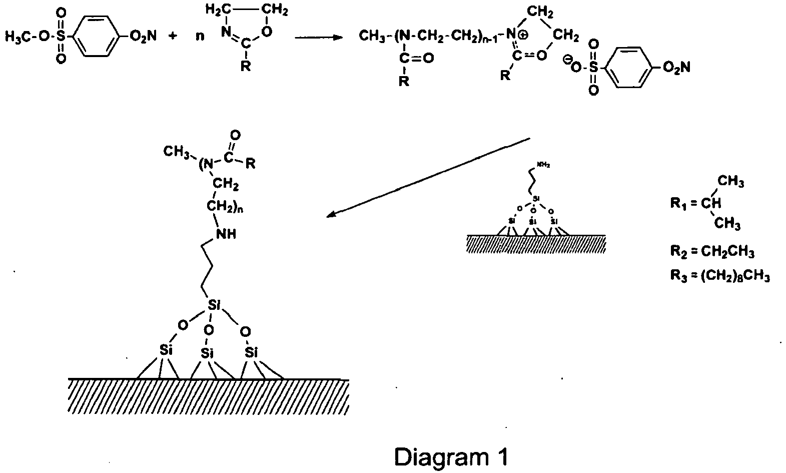

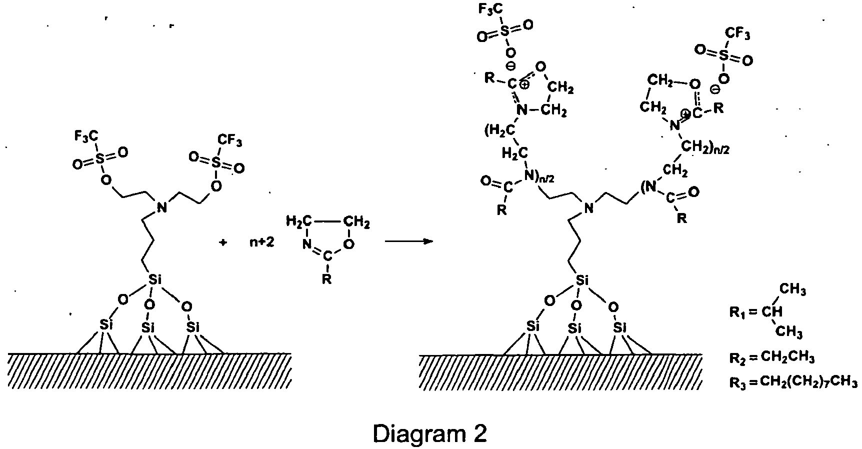

The aforementioned polymers and copolymers are biocompatible with skin cells and do not exhibit toxicity even at high (co)polymer concentrations in a culture medium. These polymers have amide groups in their polymer chain, thus they resemble peptide structures, which may influence their interaction with skin cells. Moreover, studies have found that by changing the copolymer composition, i.e., the composition of the 2-ethyl-2-oxazoline and 2-nonyl-2-oxazoline mers, the phase transition temperature (LCST) may be freely controlled from 10°C to 80°C. However, due to circumstances arising from the temperatures suitable for cell culture, this range is limited to 20°C to 37°C. PNIPAM is currently the most often used thermosensitive polymer for cell culture substrate and its grafting onto a substrate yields a layer showing a change in properties at one distinct temperature (the LCST). Copolymers of NIPAM on a surface have also been used but do not allow for a change in the phase transition temperature (LCST) in an applicable range for cell culture.

Analysis of the tests performed confirms that, as a result of the methods used for immobilization of the polymer and copolymer of 2-substituted-2-oxazoline, thermosensitive surfaces of high grafting chain density (0.1-1.8 chain/nm²) have been obtained in which the (co)polymer is covalently bonded to the base substrate by one chain end creating a so-called polymer brush on the surface.

The polymer layer produced in the exemplary design with the "grafting to" and "grafting from" methods consists of poly(2-isopropyl-2-oxazoline) or a copolymer of 2-ethyl-2-oxazoline and 2-nonyl-2-oxazoline on a glass substrate. The polymer layer reversibly changes its properties from hydrophilic to hydrophobic with ambient temperature changes in the range of 20°C to 37°C.

Substrates with a thermosensitive polymer layer have been used to assess the ability of skin cells (fibroblasts) to proliferate and create a homogeneous cell layer and subsequent cell separation in the form of a sheet without enzymatic or mechanical separation methods. The time required for cell-sheet detachment depends on the polymer and the detachment conditions and varies from several minutes to several hours, as described in following examples.

### Example 1

### 1.1. Glass substrate modification

The glass substrate, consisting of borosilicate glass plates of differing sizes (φ = 13 mm and 24×40 mm), were degreased and rinsed three times with water, then left for 1 hour in a chromic mixture (5 g sodium dichromate, 5 mL water and 90 mL concentrated sulfuric acid) and rinsed 5 times with water. Following that, the plates were left in a 1 M NaOH solution for 1 hour and rinsed 5 times with water. The wet plates were placed in a 0.1 M HCl bath for 1 hour and then water-rinsed and dampened in a 1 % acetic acid bath for 1 hour. After rinsing 5 times with water, the plates were placed in a 1 % 3-aminopropyltriethoxysilane solution with a 3 mM acetic acid solution in water. After 24 hours the plates were rinsed with a water/acetonitrile 1:1 (v:v) mixture and placed in a water bath in an ultrasonic bath for 20 seconds. Then, the plates were rinsed 5 times with water and dried under reduced pressure at room temperature. The obtained glass substrates were grafted in next stage to a poly(2-isopropyl-2-oxazoline) solution.

### 1.2. Preparation of a 2-isopropyl-2-oxazoline living polymer

In a sealed glass reactor equipped with a magnetic stirrer, 45 mL of dry acetonitrile, 7.6 g 2-isopropyl-2-oxasoline and 0.12 g p-nitrobenzene sulfuric acid as an initiator were mixed, and the solution was heated to 80°C. The reaction was left for 120 hours. After cooling, the solution was transferred under an argon atmosphere to the reactor containing the modified glass plates.

### 1.3. Immobilization

Following the reaction, the glass substrates with APTES, were left in the reactor in contact with the living 2-isopropyl-2-oxazoline polymer solution and mixed for 48 hours. Then the plates were taken out and rinsed out 3 times with water/acetonitrile 1:1 and 3 times with water and placed in ultrasonic bath for approximately 20 seconds. After additional rinsing with water, the plates were dried under reduced pressure at room temperature.

As a result of the polymer immobilization on the glass substrate, a polymer layer with a density of grafted chains of 0.8 chain/nm² was obtained.

### 1.4. Cell culture

Skin cells - fibroblasts were received during isolation from the skin fragment taken from Burn Treatment Centre (Centrum Leczenia Oparzeń) patient and after primary culture. The cells were then banked.

### 1.4.1. Preparation of the substrate for cell seeding

Glass substrates with differing plate sizes (φ = 13 mm and 24x40 mm), covered with a poly(2-isopropyl-2-oxazoline) layer, were sterilized with ethylene oxide. Afterward the substrates were placed in Petri dishes and exposed to incubation in a DMEM-ATMP culture medium at approximately 20°C for 2 hour, then placed in an incubator at 37°C and 95% humidity with an excess of 5% CO₂ for 3 hours.

The TCPS control dishes were treated in the same manner before seeding the fibroblast cells.

### 1.4.2. Cell culture and detachment

Skin cells taken from the tissue bank were plated onto substrates prepared according to the above method - 100,000 cells on a 13-mm diameter substrate and on a TCPS control dish, and 250,000 cells on a 24×40 mm substrate and on a TCPS control dish. The culture medium was replaced with fresh medium every 48 hours.

The culture was conducted until a confluence of approximately 60-70% was obtained. Then, the cells were separated from the substrate in the form of a sheet.

To separate the cells, the substrate with the cells attached was placed in an incubator in an environment that ensures a temperature of 20°C. The culture medium was replaced by a culture medium at a temperature of 20-25°C to accelerate the detachment process.

### Conclusions

Glass substrates containing a thermosensitive polymer layer of poly(2-isopropyl-2-oxazoline) covalently bounded to the substrate by the "grafting to" method provide an effective substrate for skin cell culture (photo 1). Photo 2 presents the cell culture on a TCPS control dish.

The skin cells are detached from the substrate as a sheet only after a reduction in temperature. The application of enzymatic methods for cell detachment from the substrate is not required. Fibroblasts spontaneously detach from the 13-mm diameter polymer substrate after 2 min of incubation at a reduced temperature. After 15 min to several hours, a uniform cell sheet detached from the substrate was obtained (photo 3). Cell detachment in the TCPS control dish was not observed following temperature reduction (photo 4). Fibroblasts cultured on a 24x40 mm polymer surface detached from this substrate after several hours of incubation at a reduced temperature. Cell detachment on the TCPS control dish was not observed following temperature reduction.

### Example 2

### 2.1. Glass substrate modification

Substrates of borosilicate glass plates of differing sizes (φ = 13 mm and 24x40 mm) were degreased and rinsed with water. The plates were left for 1 hours in a chromic mixture and again rinsed with water. Then the plates were left for 1 hour in a 1 M NaOH solution and rinsed again. The wet plates were placed in a 0.1 M HCl bath for 1 hour and then water-rinsed and dampened in a 1% acetic acid bath for 1 hour. After water-rinsing, the plates were placed in a 1% solution of 3-[bis(2-hydroxyethyl)amino]propyltriethoxysilane in a 3 mM acetic acid solution in water. After 24 hours the plates were rinsed with a water/acetonitrile 1:1 (v:v) mixture and placed in water in ultrasonic bath for 20 seconds, then rinsed with water and dried. The plates were then put in an air-tight reactor that had been previously blown through with argon, and a solution of 0.5 g trifluoromethanesulfonic anhydride with an addition of 0.4 g pyridine in methylene chloride was added at a temperature of approximately 0°C. The reaction was carried out at this temperature for 6 hours; then the temperature was raised to 10°C and the solution was mixed for 12 hours.

The solution was removed and the plates were rinsed three times with methylene chloride under a dry argon atmosphere and then two times with dry acetonitrile. The plates were then dried at room temperature under low pressure.

### 2.2. Polymer immobilization

The dry plates were placed in an argon atmosphere in a reactor where the solution of 2-isopropyl-2-oxazoline (6.9 g) in dry acetonitrile (450 mL) was introduced. Polymerization was performed for 220 hours at 75°C. Upon completion, the plates were placed in a solution of 2.7 g KOH in 450 mL of methanol for 24 hours at room temperature. Next, the plates were rinsed with water and dried under reduced pressure at room temperature.

As a result of the polymer immobilization on the glass substrate, a polymer layer with a density of grafted chains of 1.1 chain/nm² was obtained.

### 2.3. Cell culture

Skin cells - fibroblasts were received during isolation from the skin fragment taken from Burn Treatment Centre (Centrum Leczenia Oparzeń) patient and after primary culture. The cells were then banked.

### 2.3.1. Preparation of the substrate for cell seeding

Substrate preparation was carried out as detailed in example 1.

### 2.3.2. Cell plating onto the substrates

Cell seeding and culture were carried out as detailed in example 1.

### Conclusions

Glass substrates containing a thermosensitive polymer layer of poly(2-isopropyl-2-oxazoline) covalently bounded to the substrate by the "grafting-from" method provide an effective substrate for skin-cell culture (photo 5). Photo 6 presents the cell culture on a TCPS control dish.

The skin cells detach from the substrate as a sheet only under a reduction of temperature. The application of enzymatic methods for cell detachment from the substrate is not required.

The fibroblasts spontaneously detached from the 13-mm diameter polymer substrate after 5 min of incubation at a reduced temperature. After several hours, a uniform cell sheet detached from the substrate was obtained (photo 7). Cell detachment from the TCPS control dish was not observed following temperature reduction (photo 8). Fibroblasts cultured on a 24×40 mm polymer surface detached from the substrate after several hours of incubation at a reduced temperature.

Cells detachment from the TCPS control dish was not observed following a reduction in temperature.

### Example 3

### 3.1. Glass substrate modification

Glass substrates of varying plate sizes (φ = 13 mm and 24×40 mm) were immersed in a piranha solution for 2 hours, then rinsed three times with water and dried at 120°C for 24 hours. The cleaned plates were silylated with APTES. The plates were immersed in a 1% (v/v) APTES solution in ethanol. The reaction was carried out under a constant flow of argon for 2 hours. Then the substrates were rinsed three times in ethanol and dried at 120 °C for 24 hours. The obtained plates were placed in the reactor and exposed to grafting with a 2-ethyl-2-oxazoline and 2-nonyl-2-oxazoline living copolymer chain solution.

### 3.2. Preparation of the 2-ethyl-2-oxazoline and 2-nonyl-2-oxazoline living copolymer solution

The reactants 2-ethyl-2-oxazoline (14.7 g), 2-nonyl-2-oxazoline (3.3 g), methyl 4-nitrobenzenesulfonate (0.36 g) and acetonitrile (36 mL) were added to a separate reactor equipped with glass-teflon valve under a constant argon flow. The reactor contents were subjected to three freeze-thaw cycles. After de-freezing, the reactor was put in an oil bath and the reaction was performed at 80°C for 48 hours. After cooling, the solution was moved to the original reactor under an argon atmosphere.

### 3.3. Polymer immobilization

The solution of the 2-ethyl-2-oxazoline and 2-nonyl-2-oxazoline living copolymer was moved to the reactor containing the glass substrates that had undergone the reaction with APTES. Approximately 300 mL of dry acetonitrile was added to the reactor. The plates were left in contact with the solution, and the reactor contents were stirred for 72 hours. After the given time, the plates were removed from the reactor, rinsed three times with acetonitrile and dried at 120°C.

As a result of the polymer immobilization on the glass substrate, a polymer layer with a density of grafted chains of 0.6 chain/nm² was obtained.

### 3.4. Cell culture

Skin cells - fibroblasts were received during isolation from the skin fragment taken from Burn Treatment Centre (Centrum Leczenia Oparzeń) patient and after primary culture. The cells were then banked.

### 3.4.1. Preparation of the substrate for cell culture

The glass substrates of differing sizes (φ = 13 mm and 24x40 mm), covered with a 2-ethyl-2-oxazoline and 2-nonyl-2-oxazoline copolymer layer by the "grafting to" method, were sterilized with ethylene oxide. Afterward, the substrates were placed onto Petri dishes.

Further substrate preparation was carried out as detailed in example 1.

### 3.4.2. Cell culture and detachment

Cell seeding and culture were carried out as detailed in example 1.

### Conclusions

Glass substrates containing a thermosensitive copolymer layer of 2-ethyl-2-oxazoline and 2-nonyl-2-oxazoline covalently bonded to the substrate provide an effective substrate for skin cell culture (photo 9). Photo 10 presents the cell culture on a TCPS control dish.

The skin cells are detached from the substrate as a sheet only after a reduction in temperature. The application of enzymatic methods is not required for cell detachment from the substrate.

The fibroblasts spontaneously detach from the 13-mm and 24x40-mm sized polymer substrate after several hours of incubation at a reduced temperature (photo 11). In the case of the larger plates, extra plate shaking and careful rinsing with the medium was required to detach the cell sheet.

Cell detachment from the TCPS control dish was not observed following a reduction in temperature (photo 12).

### Patent documents quoted in the description

JP6104061, JP5192130, JP2006008975(A), EP0382214(B1), JP5192138(A), WO2010010837(A1), EP1264877A1, WO2010044417(A1), EP2348099 (A1), EP0470681(A1), US20080009063A1, WO0210349(A1), WO2010047171 (A1).

### Non-patent literature quoted in the description

1. Fishman J.A., Patience C. American Journal of Transplantation 2004, 4, 1383-1390.
2. Onons D., Cooper D.K.C., Alexander T.J.L. Xenotransplantation 2000, 7, 143-155.
3. Klama-Baryla A., Kraut M., tabut W., Maj M., Kawecki M., Nowak M., Glik J., Cichowski A., Szydto A., Lesiak M., Aniot J., Sieroń A.L. The Journal of Orthopaedics Trauma Surgery and Related Research 2011, 2(22), 77-86.
4. Han C., Zhang L., Sun J., Shi H., Zhou J., Gao C. Journal of Zhejiang University 2010, 11, 524-530.
5. Klama-Baryla A., Glik J., Kawecki M., Nowak M., Sieroń A.L. The Journal of Orthopaedics Trauma Surgery and Related Research 2009, 1, 90-99.
6. Klama-Baryla A., Glik J., Kawecki M., Nowak M., Sieroń A.L. The Journal of Orthopaedics Trauma Surgery and Related Research 2008, 3(11), 96-103.
7. Yamato M., Akiyama Y., Kobayashi J., Yang J., Kikuchi A., Okano T. Prog. Polym. Sci. 2007, 32,1123.
8. Yamato M., Utsami M., Kushida A., Konno Ch., Kikuchi A., Okano T. Tissue Eng. 2001, 7, 473.
9. Kumar P.R.A., Sreenivasan K., Kumary T.V., J. Appl. Polym. Sci. 2007, 105, 2245.
10.Nagase K., Kobayashi J., Okano T. J. R. Soc. Interface 2009, 6, 293.
11. Hutter N. A., Reitinger A., Zhang N., Steenackers M., Williams O. A., Garrido J. A., Jordan R. Phys. Chem. Chem. Phys., 2010, 12, 4360.
12.Kikuchi A., Okano T. J. Control. Release 2005, 101, 69.
13. Okano T., Yamada N., Okuhara M., Sakai H., Sakurai Y. Biomaterials 1995, 16, 297.
14.Akiyama Y., Kikuchi A., Yamato M., Okano T. Langmuir 2004, 20, 5506.
15.Tsuda Y., Kikuchi A., Yamato M., Nakao A., Sakurai Y., Umezu M., Okano T. Biomaterials, 2005, 26, 1885.
16.Tsuda Y., Kikuchi A., Yamato M., Sakurai Y., Umezu M., Okano T. J. Biomed. Mater. Res. 2004, 69A, 70.
17.Nitschke M., Götze T., Gramm S., Werner C. eXPRESS Polymer Letters 2007, 1, 660.
18.Kwon O.H., Kikuchi A., Yamato M., Okano T. Biomaterials 2003, 24, 1223.
19. Schmaljohann D. e-Polymers 2005, 021, 1.
20.Hatakeyama H., Kikuchi A., Yamato M., Okano T. Biomaterials 2005, 26, 5167.
21.Hatakeyama H., Kikuchi A., Yamato M., Okano T. Biomaterials 2006, 27, 5069.
22.Ebara M., Yamato M., Aoyagi T., Kikuchi A., Sakai K., Okano T. Biomacromolecules 2004, 5, 505.

## Claims

1. A method for making a substrate with a thermosensitive polymer layer of changeable affinity for water (LCST) within 0-80°C, in which:
i. a non-organic glass or silicon base substrate is cleaned and modified with reactive functional groups and then
ii. a thermosensitive polymer is immobilized by chain grafting and covalent bond formation in an argon protective atmosphere and in a solution,
**characterized in that** the immobilization of the thermosensitive polymer is performed from a solution of living homo- and copolymers of 2-substituted-2-oxazoline, where covalent bonds are created in the reaction between:
the nucleophilic terminating groups of the base substrate obtained as a result of substrate modification and
the cationic propagating center at the chain end of the polymers from a group of 2-substituted-2-oxazoline.

2. The method according to claim 1 **characterized in that** the immobilization is performed until a polymer layer with a brush conformation and a polymer grafting density of 0.1-1.8 chain/nm² is formed on the base substrate.

3. The method according to claim 1 or 2, **characterized by** the selection of a nucleophilic terminating group from a group including amino groups and their derivatives, hydroxyl groups and their derivatives, and thiol groups and their derivatives.

4. The method according to claim 1 or 2, **characterized by** the selection of a cationic initiating group from a group including p-toluenesulfonic acid and its derivatives with esters, trifluoromethanesulfonic acid and its derivatives with esters, alkyl halides, benzyl halides, antimony and boron fluorides, sulfuric acid and its esters, hydrochloric acid, periodic and perchloric acid and their salts, chloroformates, and trichloroacetic acid and its derivatives.

5. A method for producing the substrate with a layer of thermosensitive polymer of changeable affinity for water (LCST) within 0-80°C, in which:
i. a non-organic glass or silicon base substrate is cleaned and modified with reactive functional groups and then
ii. a thermosensitive polymer is immobilized by chain grafting with the formation of covalent bonds under an argon protective atmosphere and in a solution,
**characterized in that** the immobilization of the thermosensitive polymers from a group of homo- and copolymers of 2-substituted-2-oxazoline is performed in a solution, where covalent bonds are created during the polymerization of a monomer from a group of 2-substituted-2-oxazoline as described by formula (1) initiated by an initiator of the cationic polymerization present on the base substrate that is a result of substrate modification. 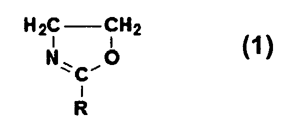 R represents a C₂-C₉ alkyl group.

6. The method according to claim 5 **characterized in that** the immobilization is performed until a polymer layer with a brush conformation and a polymer grafting density of 0.1-1.8 chain/nm² is formed on the base substrate.

7. The method according to claim 5 or 6 **characterized in that** the initiator of the cationic polymerization is selected from a group including p-toluenesulfonic acid and its derivatives with esters, trifluoromethanesulfonic acid and its derivatives with esters, alkyl halides, benzyl halides, chloroformates, and trichloroacetic acid and its derivatives.

8. The method according to claims 5 to 7 **characterized in that** the content of initiating groups in relation to at least one monomer from the group of 2-substituted-2-oxazoline group amounts from 10.0 to 0.08 mol percent.

9. A substrate consisting of a non-organic glass or silicon base substrate modified by the covalent bonding of a silyl linker through a functional group with a polymer layer immobilized onto the substrate during the grafting of the thermosensitive polymer or copolymer chains characterized- **in that**
the polymer layer is of polymer brush conformation and is represented by a polymer or copolymer from a group of 2-substituted-2-oxazoline, and the density of the chains connected to the functional group of the silyl linker is 0.1-1.8 chain/nm²

10. The substrate according to claim 9 **characterized by** a polymer layer that is covalently bonded to the functional group of a silyl linker by the element given in formula (2). 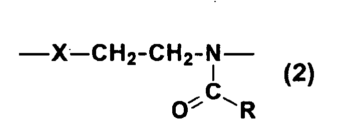 X represents a nitrogen atom, an atom of oxygen or sulfur of the silyl linker, while R represents a C₂-C₉ alkyl group of the 2-substituted-2-oxazoline monomer.

11. The substrate, according to claim 9, **characterized by** a polymer layer that is covalently bonded to the functional group of a silyl linker by the element shown in formula (3). 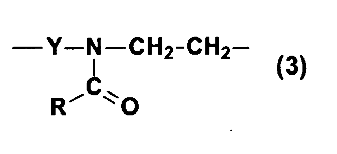 Y represents the alkyl group of the silyl linker of a linear or branched chain, while R represents a C₂-C₉ alkyl group of the 2-substituted-2-oxazoline monomer.

12. Application of the substrate with thermosensitive polymer layer made of homo- and copolymers from a group of 2-substituted-2-oxazoline as defined in claims 9 to 11, or obtained by the method defined in claims 1 to 8, for cell cultures, particularly skin cells.

## Patentansprüche

1. Verfahren zur Herstellung eines Substrats mit einer thermosensitiven Polymerschicht mit veränderbarer Affinität für Wasser (LCST) im Bereich von 0-80°C, worin:
i. ein nicht organisches Glas- oder Silizium-Basissubstrat gereinigt und mit reaktiven funktionellen Gruppen modifiziert wird, und anschließend
ii. ein thermosensitives Polymer durch Kettenpfropfung und Bildung kovalenter Bindungen in einer Argon-Schutzatmosphäre und in einer Lösung immobilisiert wird,
**dadurch gekennzeichnet, dass** die Immobilisierung des thermosensitiven Polymers aus einer Lösung aus lebenden Homo- und Copolymeren des 2-substituierten-2-Oxazolins durchgeführt wird, wobei bei der Reaktion zwischen:
den nukleophilen terminierenden Gruppen des Basissubstrats, erhalten durch Substratmodifizierung, und
dem kationischen Propagationszentrum am Kettenende der Polymere aus der Gruppe des 2-substituierten-2-Oxazolins
kovalente Bindungen gebildet werden.

2. Verfahren nach Anspruch 1, **dadurch gekennzeichnet, dass** die Immobilisierung bis zur Bildung einer Polymerschicht mit einer Bürstenkonformation und einer Polymer-Pfropfungsdichte von 0,1-1,8 Kette/nm² auf dem Basissubstrat durchgeführt wird.

3. Verfahren nach Anspruch 1 oder 2, **gekennzeichnet durch** die Auswahl einer nukleophilen terminierenden Gruppe aus der Gruppe umfassend Aminogruppen und deren Derivate, Hydroxylgruppen und deren Derivate, und Thiolgruppen und deren Derivate.

4. Verfahren nach Anspruch 1 oder 2, **gekennzeichnet durch** die Auswahl einer kationischen initüerenden Gruppe aus einer Gruppe umfassend p-Toluolsulfonsäure und deren Derivate mit Estern, Trifluormethansulfonsäure und deren Derivate mit Estern, Alkylhalide, Benzylhalide, Antimon- und Borfluoride, Schwefelsäure und deren Ester, Chlorwasserstoffsäure, Period- und Perchlorsäure und deren Salze, Chlorformiate, und Trichloressigsäure und deren Derivate.

5. Verfahren zur Herstellung des Substrats mit einer Schicht aus einem thermosensitiven Polymer mit veränderbarer Affinität für Wasser (LCST) im Bereich von 0-80°C, worin:
i. ein nicht organisches Glas- oder Silizium-Basissubstrat gereinigt und mit reaktiven funktionellen Gruppen modifiziert wird, und anschließend
ii. ein thermosensitives Polymer durch Kettenpfropfung mit Bildung kovalenter Bindungen unter einer Argon-Schutzatmosphäre und in einer Lösung immobilisiert wird,
**dadurch gekennzeichnet, dass** die Immobilisierung des thermosensitiven Polymers aus einer Gruppe von Homo- und Copolymeren des 2-substituierten-2-Oxazolins in einer Lösung durchgeführt wird, wobei während der Polymerisation eines Monomers aus der Gruppe des durch die Formel (1) beschriebenen 2-substituierten-2-Oxazolins, initiiert durch einen Initiator der kationischen Polymerisation, der auf einem Basissubstrat vorliegt, das ein Ergebnis der Substratmodifizierung ist, kovalente Bindungen gebildet werden. 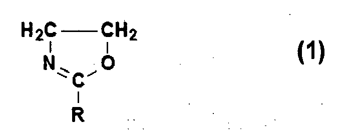 R steht für eine C₂-C₉-Alkylgruppe.

6. Verfahren nach Anspruch 5, **dadurch gekennzeichnet, dass** die Immobilisierung bis zur Bildung einer Polymerschicht mit einer Bürstenkonformation und einer Polymer-Pfropfungsdichte von 0,1-1,8 Kette/nm² auf dem Basissubstrat durchgeführt wird.

7. Verfahren nach Anspruch 5 oder 6, **dadurch gekennzeichnet, dass** der Initiator der kationischen Polymerisation aus der Gruppe umfassend p-Toluolsulfonsäure und deren Derivate mit Estern, Trifluormethansulfonsäure und deren. Derivate mit Estern, Alkylhalide, Benzylhalide, Chlorformiate, und Trichloressigsäure und deren Derivate ausgewählt ist.

8. Verfahren nach Ansprüchen 5 bis 7, **dadurch gekennzeichnet, dass** der Gehalt an initüerenden Gruppen im Verhältnis zu mindestens einem Monomer aus der Gruppe des 2-substituierten-2-Oxazolins auch von 10,0 bis 0,08 Molprozent beträgt.

9. Substrat bestehend aus einem nicht organischen Glas- oder Silizium-Basissubstrat, modifiziert durch die kovalente Bindung eines Silyl-Linkers durch eine funktionelle Gruppe mit einer auf dem Substrat während der Pfropfung der thermosensitiven Polymer- oder Copolymer-Ketten immobilisierten Polymerschischt, **dadurch gekennzeichnet, dass**:
die Polymerschicht eine Polymerbürstenkonformation aufweist und durch einen Polymer oder Copolymer aus der Gruppe des 2-substituierten-2-Oxazolins dargestellt ist, und die Dichte der mit der funktionellen Gruppe des Silyl-Linkers verbundenen Ketten 0,1-1,8 Kette/nm² beträgt.

10. Substrat nach Anspruch 9, **gekennzeichnet durch** eine Polymerschicht, die durch das in der Formel (2) angegebene Element mit der funktionellen Gruppe eines Silyl-Linkers kovalent verbunden ist. 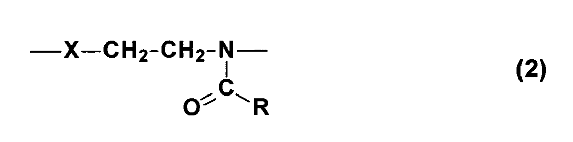 X steht für ein Stickstoffatom, ein Sauerstoff- oder Schwefelatom des Silyl-Linkers, während R für eine C₂-C₉-Alkylgruppe des 2-substituierten-2-Oxazolin-Monomers steht.

11. Substrat nach Anspruch 9, **gekennzeichnet durch** eine Polymerschicht, die durch das in der Formel (3) gezeigte Element mit der funktionellen Gruppe eines Silyl-Linkers kovalent verbunden ist. 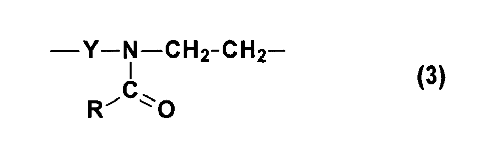 Y steht für eine Alkylgruppe des Silyl-Linkers mit einer linearen oder verzweigten Kette, während R für eine C₂-C₉-Alkylgruppe des 2-substituierten-2-Oxazolin-Monomers steht.

12. Verwendung des Substrats mit einer thermosensitiven Polymerschicht, hergestellt aus Homo- und Copolymeren aus der Gruppe des 2-substituierten-2-Oxazolins, wie in den Ansprüchen 9 bis 11 definiert, oder erhalten durch das in den Ansprüchen 1 bis 8 definierte Verfahren, für Zellkulturen, insbesondere Hautzellen.

## Revendications

1. Un procédé de fabrication d'un substrat avec une couche de polymère thermosensible d'affinité variable pour l'eau (TCIS) dans la plage de 0-80°C, dans lequel :
i. un substrat non-organique de verre ou à base de silicium est nettoyé et modifié par des groupes fonctionnels réactifs, et ensuite
ii. un polymère thermosensible est immobilisé par greffage de chaîne et formation de liaisons covalentes dans une atmosphère protectrice d'argon et dans une solution,
**caractérisé en ce que** l'immobilisation du polymère thermosensible est réalisée à partir d'une solution d'homo- et copolymères vivants de 2-oxazoline substituée en 2, où liaisons covalentes sont créées dans la réaction entre:
les groupes terminaux nucléophiles du substrat de base obtenu à la suite de la modification du substrat et
le centre de propagation cationique à l'extrémité de chaîne des polymères d'un groupe de 2-oxazoline substituée en 2.

2. Le procédé selon la revendication 1, **caractérisé en ce que** l'immobilisation est réalisée jusqu'à ce qu'une couche de polymère avec une conformation en brosse et une densité de greffage de polymère de 0,1-1,8 chaîne/nm² se forme sur le substrat de base.

3. Le procédé selon la revendication 1 ou 2, **caractérisé par** la sélection d'un groupe terminal nucléophile à partir d'un groupe comprenant des groupes amino et leurs dérivés, des groupes hydroxy et leurs dérivés, et des groupes thiols et leurs dérivés.

4. Le procédé selon la revendication 1 ou 2, **caractérisé par** la sélection d'un groupe initiateur cationique à partir d'un groupe comprenant acide paratoluènesulfonique et ses dérivés avec des esters, acide trifluorométhanesulfonique et ses dérivés avec des esters, halogénures d'alkyle, halogénures de benzyle, fluorures d'antimoine et de bore, acide sulfurique et ses esters, acide chlorhydrique, acide périodique et perchlorique et leurs sels, chloroformiates et acide trichloroacétique et ses dérivés.

5. Un procédé de production du substrat avec une couche de polymère thermosensible d'affinité variable pour l'eau (TCIS) dans la plage de 0-80°C, dans lequel :
i. un substrat non-organique de verre ou à base de silicium est nettoyé et modifié par des groupes fonctionnels réactifs, et ensuite
ii. un polymère thermosensible est immobilisé par greffage de chaîne avec la formation de liaisons covalentes sous une atmosphère protectrice d'argon et dans une solution,
**caractérisé en ce que** l'immobilisation des polymères thermosensibles d'un groupe d'homo- et copolymères de 2-oxazoline substituée en 2 est réalisée dans une solution, où liaisons covalentes sont créées lors de la polymérisation d'un monomère d'un groupe de 2-oxazoline substituée en 2 comme décrit par la formule (1), initiée par un initiateur de la polymérisation cationique présente sur le substrat de base qui est le résultat de la modification du substrat. 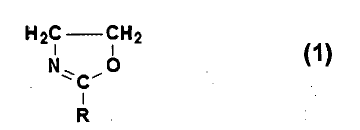 R représente un groupe alkyle C₂-C₉.

6. Le procédé selon la revendication 5, **caractérisé en ce que** l'immobilisation est réalisée jusqu'à ce qu'une couche de polymère avec une conformation en brosse et une densité de greffage de polymère de 0,1-1,8 chaîne/nm² se forme sur le substrat de base.

7. Le procédé selon la revendication 5 ou 6, **caractérisé en ce que** l'initiateur de la polymérisation cationique est choisi à partir d'un groupe comprenant acide paratoluènesulfonique et ses dérivés avec des esters, acide trifluorométhanesulfonique et ses dérivés avec des esters, halogénures d'alkyle, halogénures de benzyle, chloroformiates et acide trichloroacétique et ses dérivés.

8. Le procédé selon les revendications 5 à 7 **caractérisé en ce que** le contenu en groupes initiateurs par rapport à au moins un monomère du groupe de 2-oxazoline substituée en 2 s'élève aussi de 10,0 à 0,08 pour cent en moles.

9. Un substrat constitué par un substrat non-organique de verre ou à base de silicium modifié par la liaison covalente d'un lieur à base de silyle par l'intermédiaire d'un groupe fonctionnel avec une couche de polymère immobilisée sur le substrat lors du greffage des chaînes de polymère thermosensible ou copolymère, **caractérisé en ce que** :
la couche de polymère est de conformation polymérique en brosse et est représentée par un polymère ou copolymère d'un groupe de 2-oxazoline substituée en 2, et la densité des chaînes reliées au groupe fonctionnel du lieur à base de silyle est 0,1-1,8 chaîne/nm².

10. Le substrat selon la revendication 9 **caractérisé par** une couche de polymère qui est reliée de manière covalente au groupe fonctionnel d'un lieur à base de silyle par l'élément donné dans la formule (2). 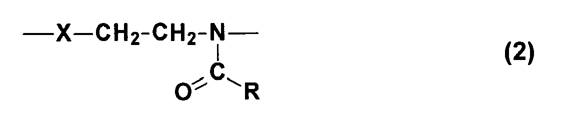 X représente un atome d'azote, un atome d'oxygène ou de soufre du lieur à base de silyle, tandis que R représente un groupe alkyle C₂-C₉ du monomère de 2-oxazoline substituée en 2.

11. Le substrat selon la revendication 9 **caractérisé par** une couche de polymère qui est reliée de manière covalente au groupe fonctionnel d'un lieur à base de silyle par l'élément indiqué dans la formule (3). 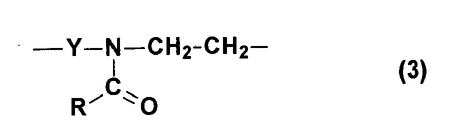 Y représente le groupe alkyle du lieur à base de silyle d'une chaîne linéaire ou ramifiée, tandis que R représente un groupe alkyle C₂-C₉ du monomère de 2-oxazoline substituée en 2.

12. Application du substrat avec une couche de polymère thermosensible fait de homo- et copolymères d'un groupe de 2-oxazoline substituée en 2, tel que défini dans les revendications 9 à 11, ou obtenu par le procédé défini dans les revendications 1 à 8, pour les cultures cellulaires, en particulier cellules cutanées.
